(19) Europäisches Patentamt
European Patent Office
Office européen des brevets

(11) Publication number: **0 492 525 A2**

(12) **EUROPEAN PATENT APPLICATION**

(21) Application number: **91121990.5**

(22) Date of filing: **20.12.91**

(51) Int. Cl.⁵: **C12P 21/08**, C12N 9/50, C12N 15/30, A61K 39/018

(30) Priority: **21.12.90 AU 4051/90**

(43) Date of publication of application: **01.07.92 Bulletin 92/27**

(84) Designated Contracting States: **AT BE CH DE DK ES FR GB GR IT LI LU MC NL SE**

(71) Applicant: **COMMONWEALTH SCIENTIFIC AND INDUSTRIAL RESEARCH ORGANIZATION**
**14 Limestone Avenue**
**Canberra, Australian Capital Territory 2601(AU)**

(72) Inventor: **Casu, Rosanne Elena**
**19 Mackie Road**
**Narangba, Oueensland 4504(AU)**
Inventor: **Commins, Margarete Anne**
**32 Cameron Parade**
**Wynnum, Oueensland 4178(AU)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **Babesial protease antigen.**

(57) Antigens inducing protective immunity against homologous or heterologous challenge with Babesia bovis which include an antigen which is immunoreactive with a monoclonal antibody recognising a protein located within rhoptries organelles at the apical end of Babesia bovis in infected erythrocytes. Recombinant antigens are also claimed and in particular a recombinant antigen which is a component of 21B4 antigen of 313 base pairs and disclosed in FIG 11. Processes for preparation of monoclonal antibody T21B4 as well as processes for preparation of the above antigens are also claimed. Vaccines including the antigens in combination with an appropriate adjuvant are also claimed.

EP 0 492 525 A2

THIS INVENTION relates to a babesial bovis antigen and associated vaccine which may be effective in providing immunity in relation to babesiosis against different strains of Babesia concerning both heterologous and homologous challenge.

In Australian Patent Specification 581930 there is described a process for preparation of a native babesial antigen which was prepared from a lysate of Babesia infected erythrocytes. The lysate was purified by removal of contaminants including oxyhaemoglobin and a protease fraction was obtained from the purified lysate by gradient gel electrophoresis under denaturing conditions in the presence of a protease substrate.

It has now been ascertained that the native babesial antigen was relatively impure containing not only the protease antigen but also other contaminant proteins which were capable of producing undesirable side reactions, Also when the process of patent 581930 was carried out a number of times often different end materials would result which showed that the babesial protease antigen was not obtained in a pure state and thus could not be used effectively as a commercial vaccine.

Reference may also be made to Suarez et al to a publication entitled "Characterisation of the Gene Encoding a 60kD Babesia bovis Merozoite Protein with Conserved and Surface Exposed Epitopes" by C Suarez, G H Palmer, D Jasmer, S.A. Hines and L Perryman of Washington State University and T F McElwain of University of Florida. This document was published on November 7 1990 at the Veterinary Hemoparasite Research Workers Meet in Chicago USA.

The abovementioned reference is noteworthy in relation to the present invention in that it identified a 60kD merozite surface polypeptide. It was further discovered that the gene which encodes the entire amino acid sequence of the polypeptide had 1990 base pairs and the polypeptide was considered to be protective if included in a vaccine.

The abovementioned Suarez et al article therefore appears to have published a gene structure which corresponds to a gene structure referred to in this invention which encodes a polypeptide hereinafter called 21B4 antigen. However in accordance with the present invention there have now been identified various inserts or components of the 21B4 gene which when expressed as corresponding polypeptides have been found to provide immunity in relation to B. bovis infection in cattle against different strains of Babesia concerning both heterologous and homologous challenge.

It therefore is an object of the present invention to provide a 21B4 antigen in recombinant or native form as wall as components of the 21B4 antigen gene which upon expression provides corresponding polypeptides which are immunogenic and thus may form the basis of a commercial vaccine for treatment of babesiosis and which also may be protective against babesiosis.

It is a further object of the invention to provide monoclonal antibodies which may correspond to the 21B4 antigen.

A further object of the invention is to provide processes for preparation of the abovementioned antigens.

The present invention therefore in one aspect provides a process for preparation of a monoclonal antibody T21B4 antigen which includes the following steps:

(i) fusing mouse splenocytes with an antigenic fraction derived from a lysate of Babesia bovis infected erythrocytes which have been subjected to further purification to remove contaminants including oxyhaemoglobin and from which a protease fraction has been isolated following sodium dodecyl sulphate electrophoresis;

(ii) screening hybridomas obtained after step (i) with infected erythrocytes to locate hybridomas which stained the parasite in infected cells and thereafter obtaining a monoclonal antibody of isotype $IgG_2b$; and

(iii) purifying the monoclonal antibody extract of interest by passing through a column which binds to isotype $IgG_2b$ followed by subsequent elution.

The starting material referred to in step (i) is prepared as described in detail in Australian Patent 581930 or as described in Commins et al (1985) Int. J. Parasitol; 15: 491-495.

The hybridoma cells in step (ii) may be diluted out in any suitable medium such as RPMI1640 supplemented with depleted foetal calf serum and the diluted cells placed in for example a microlitre plate having a plurality of walls. Any suitable screening assay may be used to locate hybridomas of interest and in this regard hybridoma supernatants may be screened by ELISA, IFA, Western blotting or other appropriate assay suitably using B. bovis infected erythrocytes. Positive wells may be cloned and recloned by limiting dilution. The clone designated 21B4 was selected by an IFA technique wherein the 21B4 clone stained the infected erythrocyte preferentially with only minor staining of the parasite and with no staining of uninfected erythrocytes. A selection was made on the basis that antibody against the infected erythrocyte should be more effective than antibody against the parasite itself since the parasite and its antigenic epitopes are masked by virtue of the fact that the parasite resides within the infected erythrocyte.

The binding of monoclonal antibody 21B4 is characterised by IFA and IMMUNOGOLD staining as described hereinafter wherein granular staining of organelles at the apical end of B. bovis in infected erythrocytes was indicated. Such organelles are indicated hereinafter as rhoptries.

In this specific case the monoclonal antibody 21B4 was found to be of $IgG_2$ isotype and this was found to be beneficial because of stability and case of purification.

Once the desired monoclonal antibody was then identified it was then purified in appropriate manner such as by harvesting supernatant fractions from selected cells after centrifugation and concentration of the supernatant fractions by dialysis against polyethylene glycol. The concentrate was then passed through a column which could bind the relevant isotype. In the case of $IgG_2$ this was found to be Protein A which could bind to a suitable support such as agarose beeds, acrylic, cellulosic or nitrocellulose supports. Preferably Sepharose and in particular Sepharose 4B was found to be suitable. Bound protein was eluted with any suitable elutant of low pH (eg pH3.0) which may comprise glycine - HCl of pH 3.0, acetate buffer salts of relatively high concentration (eg 2-5M NaCl) or urea.

In another aspect the invention provides a method of preparation of native antigen 21B4 which includes the steps of:

(i) passing lysed erythrocytes previously infected with B. bovis through an affinity column to which 21B4 monoclonal antibody was previously bound; and

(ii) eluting the 21B4 antigen from the column;

The 21B4 antigen may be prepared from a crude mixture of soluble babesial antigens obtained from infected erythrocytes or alternatively lysed infected erythrocytes by any suitable means known in the art of affinity purification. For example the antigen may be purified by immunoadsorption onto the monoclonal antibody mounted in a suitable support such as agarose beads, acrylic, cellulosic or nitrocellulosic supports. The monoclonal antibody may be coupled by ligands (eg protein A) to the support. The supported antibody may be packed in an affinity chromatography column and equilibrated with a suitable buffer. Optionally the column may also be treated to inactivate any non-specific binding sites. The crude mixture of soluble babesial antigens may be passed through the column to affect binding of the antigen specified by the monoclonal antibody. The bound antigen may be eluted from the column using a suitable elution buffer such as those discussed above in relation to preparation of the monoclonal antibody.

The invention also comprises a method of preparation of DNA fragments which encode protective component(s) of the 21B4 antigen and corresponding polypeptides which includes the steps of:

(i) screening a cDNA B. bovis library or genomic library with T21B4 monoclonal antibody;

(ii) identifying positive clone(s);

(iii) isolating the DNA corresponding to said positive clone(s);

(iv) preparing oligonucleotide(s) corresponding to selected DNA sequences of the DNA in step (iii);

(v) screening a B bovis cDNA or genomic library with the oligonucleotide probe(s) prepared in step (iv);

(vi) identifying positive clone(s); and

(vii) isolating the DNA corresponding to the positive clone(s).

The invention also includes within its scope a method of preparing a gene which encodes a recombinant 21B4 antigen which may include steps (i), (ii) and (iii) above as well as the recombinant antigen per se.

In step (i) above a suitable cDNA library was λgt 11 library derived from B. bovis. Positive clones were identified by ELISA although other suitable methods such as RIA or IFA could be utilised.

Preferably in step (iv) oligonucleotides were prepared which corresponded to the two end DNA sequences of a positive clone formed in step (iii). The cDNA library screened in step (v) is suitably a λgt 10 library derived from B. bovis.

It was found that the DNA sequence elucidated in step (iii) also corresponded to the DNA sequence of a clone isolated in step (vii). Other clones were isolated in step (vii) also corresponded to antigen components of 21B4 antigen which here found to be protective after vaccination trials.

The invention also includes within its scope the oligonucleotide probe(s) identified hereinafter.

Preferably in step (iv) the positive clones were subcloned into an expression vector such as pGGM or pUC for sequencing purposes. Clones of interest were also subcloned into pGEX or other suitable vector for expression purposes.

It will also be appreciated that genomic libraries could also be used in steps (i) or (v) above as well as cDNA libraries although the latter may be preferred in isolating DNA fragments. It was also noted that the relevant DNA sequences which were elucidated did not appear to contain any introns.

MATERIALS AND METHODS

CHEMICALS

All chemicals were of analytical grade unless otherwise stated. Electrophoresis chemicals and marker proteins were from Bio-Rad, LKB, BRL or Amersham Int.

1. **PREPARATION OF MONOCLONAL ANTIBODIES.**

Monoclonal antibodies were prepared by the fusion of the splenocytes from BALB/c mice immunised with the protease containing fraction, which has previously been shown to be protective (Commins et al., 1985, Int. J. Parasitol., 15: 491-495 and Australian patent no. 581930), with the mouse P3x63 myeloma cells using standard hybridoma culture procedures (Wright et al., 1983, Infection and Immunity, 41:241-250). A number of positive hybridomas were obtained. The monoclonal antibodies secreted by a number of these hybridomas were screened by IFA and isotyped (Table 1). T21B4 monoclonal antibody was chosen for further study because it specifically stained the parasite as well as being isotype $IgG_2b$ (igG-isotype antibodies are very stable for linkage to affinity supports). Native material affinity-purified using this monoclonal antibody was found to be protective in a preliminary vaccination trial (data not shown). The hybridoma cells were propagated in RPMI 1640 medium containing 10% foetal calf serum. The antibody was harvested from the secreting cells by centrifugation at 3000 x g for 10 mins. Antibody was purified from culture supernatant essentially by the method of Underwood et al. (1983, J. Immunological Methods, 60:33-45). Approximately 9 l of supernatant containing 10 mM axide, was concentrated to four lots of approximately 200 ml by reverse dialysis against polyethylene glycol (PEG) 20000. This is a gentle method of concentration which promotes maximum yields of antibody, but other possible methods of concentration which promotes maximum yields of antibody, but other possible methods of concentration include pressure dialysis using e.g. an amicon UM10 filter (molecular weight cut-off 10 000 Da). The concentrate was equilibrated against phosphate-buffered saline (PBS) and allowed to recirculate overnight on a column of Protein A Sepharose (1.6 x 11.5 cm, flow rate 10 ml/h). Ammonium sulphate could also have been used to precipitate the IgG but Protein A Sepharose is preferred as a more gentle method. A rabbit anti-mouse IgG affinity column could also be used to achieve the same result. The column was washed extensively with PBS. Bound protein was eluted with 0.1 M glycine-HCL, pH 3.0 into tubes containing sufficient saturated Tris solution to neutralise each fraction. Other possible elutants are 2-5M NaCl or 2M urea. The column was re-equilibrated and the wash fraction extracted twice for antibody. The eluted fractions were pooled, concentrated by vacuum dialysis, and dialysed against 0.1 M $NaHCO_3$ containing 0.5 M NaCl to neutralise the solution and aid stability. Protein concentration was determined using "Protein Assay Kit" (Bio-Rad).

2. **COUPLING OF T21B4 MONOCLONAL ANTIBODY TO THE COLUMN.**

The purified antibody was coupled to swollen CNBr activated-Sepharose 4B (Pharmacia) following the manufacturer's instructions. The coupling reaction was followed by monitoring the removal of protein from the supernatant of a centrifuged aliquot. After an estimated 90% coupling (70 min) the reaction was stopped by blocking unreacted sites with 100 mM Tris, pH 8.0 and the gel further washed as per instructions. The antibody coupled gel was packed into a column (1.6 x 4.5 cm) and equilibrated in PBS, 10 mM sodium aside, pH 7.4. Just prior to use the column was washed with the above buffer, without azide, followed by freshly prepared 0.1 M glycine pH 3.0, then re-equilibrated with starting buffer.

3. **PREPARATION OF 21B4 ANTIGEN.**

(a) Growth, harvesting, concentration and lysis of cells.

B. bovis (Samford) infected erythrocytes were harvested from spelectomised calves and concentrated as described previously (Mahoney, D.J., Experimental Parasitology, 20:301-341, 1967). The 100% infected intact red blood cells were lysed in 5 volumes of cold distilled water and spun at 10,000 g for 60 min at 4°C. The supernatant lysate was passed through a column of CM Sephadex A50 (Pharmacia) pre-equilibrated with 0.01 M phosphate buffer pH 5.5 (Goodger, Wright and Waltisbuhl, 1983, Z. Parasitenkd., 69: 473-482). The breakthrough fraction was dialysed against PBS and concentrated back to the starting volume using PEG.

(b) APPLICATION TO AFFINITY COLUMN.

After concentration, the sample fraction was diluted with an equal volume of 150 mM NaCl, pH 7.4 (affinity column running buffer) and the sample allowed to cycle at 50 ml/h through columns of Sepharose 4B. The columns were washed with 200 ml of affinity colum n running buffer, the monoclonal antibody affinity column disconnected, and washed with 2 M NaCl, 0.1% Tween, 0.1 M boric acid, 0.025 M sodium tetraborate (200ml).

(c) Elution from affinity column.

antigen was eluted from the column with 0.1 M glycine pH 3.0 in 2 ml fractions and neutralised using saturated Tris solution. The eluate was concentrated to approximately the starting volume using PEG 20000.

4. **ANALYSIS OF ELUATE**

(a) Western blot.

Substrate SDS electrophoresis of protein samples throughout the purification was performed in gelatin impregnated acrylamide as described by Heussen and Dowdle (1980, Analytical Biochemistry, 102: 196-202), but without plasminogen. Attempts at immunoblotting were unsuccessful as the antigen would not bind to the nitrocellulose. Material eluted from the T21B4 affinity column and run on gelatin containing gels showed a broad band of proteolysis at 68-70 kDa (Figure 1). Silver staining of this material in the absence of gelatin showed a fuzzy band of this size (Figure 2). Use of 2-mercaptoethanol destroyed protease activity.

(b) ELISA.

Positive reactions were obtained using ELISA with the affinity purified protein. The T21B4 monoclonal antibody was diluted to 1:10 and goat anti-mouse IgG-peroxidase labelled conjugate (KPL) was diluted to 1:500.

(c) Immunogold.

The binding of the T21B4 monoclonal antibody was examined by immunofluorescent antibody testing (IFA) and immunogold localisation. The T21B4 monoclonal antibody indicated granular staining of the apical end of B. bovis in infected erythrocytes by IFA (Figure 3). Immunogold localisation using 9 nm particles of gold conjugated to protein A showed staining of organelles at the apical end of pairs of parasites which are typically found in brain tissue of acutely infected cattle. The morphology and location of these organesses was consistent with that of rhoptries (Figure 4). Rhoptires are involved in the secretion of materials (including proteins) that enable the parasite to enter and leave erythrocytes.

5. **PRODUCTION OF ANTISERA AGAINST AFFINITY PURIFIED 21B4 ANTIGEN.**

(a) Bovine antiserum

Three months old Bos taurus British Breed steers were purchased from an area known to be free from Boophilus microplus, the tick vector for B. bovis in Australia. The sera of the animals were then screened by both ELISA and IFA to determine that they contained no detectable antibodies to either Babesia species.

Four steers received 10 µg of affinity purified native 21B4 antigen in 1 ml of normal saline and 1 ml of Freunds Complete Adjuvant (FCA) as a water-in-oil emulsion by subcutaneous injection on day 0 and 28. Serum samples were taken on day 56 and tested by both ELISA and IFA for antibodies. The mean IFA titre on day 56 was 1:100 and the staining was similar to that seen with the monoclonal antibody T21B4. This staining was punctate, being located in the apical pole of the parasites. ELISA titres with native antigen (Waltisbuhl et al 1987, Parasitology Research, 73: 126-131) were weakly positive with all vaccinated animals.

As well, the IgG was isolated from the bovine antisera and coupled to CNBr activated-Sepharose 4B (as described above) which was reacted with the lysate from infected erythrocytes. After appropriate washings and elution (as described above) an eluate was obtained which, on SDS substrate electrophoresis, produced a typical lysis band at 70 kDa (data not shown).

(b) Rabbit antiserum.

Rabbit antiserum to affinity purified 21B4 antigen was produced by the procedure of Gell and Coombs (1963, Clinical Aspects of Immunology, 1st edition, p7, Blackwell, Oxford). The IFA status of the rabbit antisera was identical to that of the T21B4 monoclonal antibody. Likewise, affinity chromatography as per the bovine antiserum yeilded an eluate which had identical substrate electrophoretic properties.

6. **SCREENING OF B. BOVIS cDNA LIBRARY WITH THE T21B4 MONOCLONAL ANTIBODY.**

(a) Identification of clones.

A random-primed B. bovis (Samford attenuated line) cDNA lambda gt11 library (provided by Dr. K R Gale, described in Australian Patent specification 48634/90, "Polypeptide, antigens or vaccines protective against Babesiosis") was screened with the T21B4 monoclonal antibody using standard immunoscreening techniques. 120,000 recombinants of the lambda gt11 library were plated out on 6 large petri dishes using Y1090 plating cells. Plaques were allowed to grow at 42°C for 3.5h, copies of the plaques were made by transferring to duplicate discs of Hybond N (Amersham Int.) soaked in 10 mM IPTG (isopropyl-$\beta$-D-thiogalactopyranoside). The plates and the filters were marked with India ink and a needle and incubated at 37°C for 2h. The filters were removed and incubated for at least 30 min at room temperature in PBS containing 10% horse serum. The filters were then incubated for 3 h, with undiluted culture supernatant containing T21B4 monoclonal antibody. The filters were washed 3 x 15 min at room temperature in PBS containing 0.2% Tween-20. The filters were incubated as above for 2 h with horseradish peroxidase-conjugated bovine anti-mouse IgG (KPL) diluted 1:500. The filters were washed 3 x 5 min at room temperature with PBS. Positives were identified by developing the filters in 5.6 mM 4-chloro-1-napthol, 0.01% (v/v) $H_2O_2$ in 50 mM Tris HCl pH 8.2. The positive plaques were identified on the original plates and harvested into 500 $\mu$l of SM (100 mM NaCl, 8 mM $MgSO_4$, 50 mM Tris. HCl pH 7.4). After two further rounds of purification one positive clone, designated B. bovis lambda gt11 1.1, was identified.

A lysogen was made in E. coli Y1089 to give a $\beta$-galactosidase fusion protein using standard techniques. A culture of Y1089 at $OD_{600}$ -1 was harvested by centrifugation and resuspended to $OD_{600}$ 3.5. To 100 $\mu$l of cells were added sufficient phage corresponding to the positive clone above for a multiplicity of infection of 20 per bacterial cell. The cells were incubated for 30 min at 30°C. Twelve colonies were replica plated in duplicate. One plate was incubated at 32°C and the other at 42°C. Temperature sensitive colonies were lysogens of the recombinant phage. A colony of the lysogen was inoculated into 50 ml LB broth containing ampicillin and incubated at 32°C to $OD_{600}$-0.5 IPTG was added to 0.1 mM and the culture was shaken at 42°C for 20 min and at 30°C for 40 min. Cells were pelleted by centrifigation and resuspended in 25ml of 1 mM EDTA, 1 mM DTT. The cells were lysed by a freeze-thaw cycle in liquid nitrogen followed by sonication (B. Braun Labsonic 1510). The lysate was centrifuged at 12,000 g for 15 min at 4°C. The proteins in a sample of thye supernatant were separated by SDS-PAGE according to the method of Laemmli (1970, Nature, 227: 680-685). Proteins were transferred to nitrocellulose membranes according to the method of Towbin et al. (1979, Proc. Natl. Acad. Sci. USA, 76: 4350-4354). The T21B4 monoclonal antibody bound to the $\beta$-galactosidase fusion protein on a Western blot (Figure 5).

(b) Preparation of phage DNA

A single plaque of phage B. bovis lambda gt11 1.1 was picked and resuspended in 500 $\mu$l of the phage suspension were plated on a large plate of LB medium using Y1090 cells and grown until confluent lysis had been obtained. The plate was then overlayed with 12ml of cold SM and stored in the refrigerator overnight to allow the phage to diffuse into the medium. The medium was recovered with a sterile pipette and centrifuged for 10 min at 12,000 g at 4°C. To the clarified supernatant was added an equal volume of 2 M NaCl containing PEG 6000 (20% w/v), and then mixed thoroughly. The mixture was allowed to sit on ice for 60 min, and then centrifuged for 20 min at 12,000 g at 4°C to pellet the phage. The supernatant was decanted and the tube was allowed to drain thoroughly before the pellet was resuspended in 3 ml of SM. An equal volume of chloroform was added and the tube was vortexed for 1 min and then centrifiged for 10 min at 3,000 g at 4°C. The supernatant was transferred to an ultracentrifuge tube and 0.71 g of CsCl per ml of supernatant was added. The tube was centrifuged for 5 h at 80,000 rpm at 20°C in a Beckman TLA100 rotor. The phage band was collected with a needle and the DNA was extracted using the formamide method of Davis, Botstein and Roth (A Manual for Genetic Engineering: Advanced Bacterial Genetics, Cold Spring Harbor, 1980). The DNA was analysed by digesting 5 $\mu$l of the dissolved DNA with restriction enzymes and electrophoresing through an agarose gel with DNA size markers.

(c) Sequencing of the cDNA insert.

The EcoRi insert contained in this clone was subcloned into pGEM7Zf(+) (Promega Corp.) for sequencing. Escherichia coli strain JM83 was made competent using $CaCl_2$. Positive clones ware identified on media containing ampicillin, 5-borom-4-chloro-3-indolyl-β-D-glaactoside (X-gal) and IPTG by the absence of a blue colour. Clones were toothpicked into LB medium containing ampicillin and grown overnight with shaking at 37° C. DNA was prepared from these cultures by the method of Birnboim and Doly as described in Maniatis, Fritsch and Sambrook (Molecular cloning: A laboratory manual, Cold Spring Harbor, 1982). The DNA was prepared for sequencing by denaturing with NaOH, neutralising and ethanol precipitating. The pellet was redissolved in 6 μl of ditilled water, 3μl of sequencing primer (10mg/ml) corresponding to either the T7 RNA polymerase promoter sequence or the SP6 RNA polymerase promoter sequence, and 1 μl of 10 x Klenow buffer (100 mM Tris HCl, pH7.4, 500 mM NaCl). The dissolved DNA was equilibrated at 37° C for at least an hour. The DNA was sequenced using the dideoxy method of Sanger et al. (1977, Proc. Natl. Acad. Sci. USA, 74: 5463-5467) using the reagents and procedures of Promega Corp. The EcoRI insert in the plasmid designated pT#1 was completely sequenced (Figure 6, Seq ID No. 1). When the DNA sequence was translated (Figure 6) an open reading frame, in frame with the vector β-galactosidase gene, was detected that ran all the way through the insert.

7. **ISOLATION OF FURTHER cDNA CLONES OF 21B4.**

(a) Use of synthetic oligonucleotides.

Oligonucleotides ware synthesised on a Pharmacia LKB Gene Assembler or Gene Assembler Plus. Two 20-mers corresponding to the two ends of the pT#1 EcoRI insert, designated 21B4.1 and 21B4.2 (Figure 7), were synthesised. All Southern blots and screens of B. bovis libraries using the 21B4.1 and 21B4.2 oligonucleotides were performed under the following conditions;

DNA was transferred to Hybond N or N+ filters (Amersham Int.) as described above or by Southern blotting as described in Maniatis, Fritsch and Sambrook (Molecular cloning: A laboraoty manual, Cold Spring Harbor, 1982). The filter-bound DNA was denatured with 0.5 M NaOH, 1.5 M NaCl; followed by neutralisation with 0.5 M Tris, 1.5 M NaOH, 1.5 M NaCl; followed by neutralisation with 0.5 M Tris, 1.5 M NaCl and rinsing in 2 x SSC. The filters were air dried briefly and exposed, DNA side down, to a transilluminator (256 nm) for 3.5 min or placed on 3MM filter paper soaked with 0.4 M NaOH for 20 min.

The probe was prepared by end-labelling 100-200 ng of the oligonucleotide with γ-32P-ATP. Filters were prehybridised in 6 x SSC, 5 x Denhardt's solution, 50μg/ml herring sperm DNA for four hours at 59° C, then placed in fresh solution with the probe and hybridixed at 59° C overnight. The filters were subsequently washed in 6 x SSC, 0.1% SDS for 3 x 15 minutes at 59° C, and autoradiographed.

(b) Isolation of further cDNA clones containing the 21B4 gene.

The T21B4.2 oligonucleotide was used to screen a B. bovis (Samford 4 passages) cDNA lambda gt10 library (provided by Dr. P. W. Riddles, described in Australian patent application no. 61249/90). The screen of the cDNA library yeilded two independent cDNA clones, designated II 1.1.1.1 and V 1.1.1.1. All of the fragments from clone V 1.1.1.1. were subcloned into pGEM7Zf(+) for sequencing (see Figure 8). The isnert from pT#13 was sequenced at both ends as well as partial sequence internally and is approx. 1200 bp in length (Figure 9, T7 end Seq ID No. 2 and Figure 10, central region Seq ID No. 3). No differences were found between the sequence of pT#1 and the equivalent region of pT#13 (data not shown). Several regions of open reading frames have been identified (Figures 9 and 10). The inserts from pT#10 and pT#5 were completely sequenced and are 484 bp in length (Figure 11, Seq ID No. 4). An open reading frame of 163 amino acids was identified from the DNA sequence of pT#10 and pT#5 (Figure 11).

8. **PRODUCTION OF GLUTATHIONE-S-TRANSFERASE-21B4 FUSION PROTEINS**

The 166 bp EcoRI fragment from pT#1, the 313 bp EcoRI fragment from pT#5 (Figure 12) and the -1200 bp EcoRI fragment from pT#13 (data not shown) were recloned into pGEX expression vectors to produced glutathione-S-transferase fusion proteins:
pGEX-1:166 bp to give pX167 to give pX309.

pGEX-2T:1200 bp to give pXT1200.

Fusion proteins were produced and purified by a method adapted from Smith and Johnson (1988, Gene, 67: 31-40). Five mlo of an overnight culture of E. coli SL-1 (pX167, pX309) transformed with the appropriate recombinant plasmid was subcultured into 250 ml LB medium and grown with vigorous shaking for seven hours at 37° C. Production of the fusion protein was induced by the addition of IPTG to 1 mM, with growth continuing with vigorous shaking overnight. The cells were pelleted and resuspended in 5 ml of PBS and lysed on ice by sonication for 3 x 30 seconds at 100 watts (B Braun Labsonic 1510). The insoluble material was pelleted and the supernatant removed to a fresh tube and mixed with 8 ml of 50% glutathione-agarose beads in PBS. The beads were gently mixed with the fusion protein by inversion for ten minutes, then collected by brief low speed centrifugation. The supernatant was removed and kept, and the beads were washed three times with 50ml PBS. The fusion proteins were eluted from the beads by competition with free glutathione using 2 x 5 min wahses with 8 ml of 50 mM Tris.HCl (pH8.0) containing 5mM reduced glutathione (freshly prepared). If necessary, the beads were washed with 3 x 50 ml PBS to remvoe the reduced glutathion and the bead supernatant was reapplied and the process repeated. This increased the yield. The purified fusion proteins were then run on SDS-PAGE gels and stained with Coommassie Blue R-250 (Figure 12).

The ability of fusion proteins encoded by pX167, pX309 and pXT1200, designated 21B4-167, 21B4-309 and 21B4-1200 respectively, to bind T21B4 monoclonal antibody was then ascertained. The T21B4 monoclonal antibody bound to the 21B4-167 and 21B4-1200 fusion proteins, it did not bind to 21B4-309 fusion protein (data not shown).

## 9. **VACCINATION AND CHALLENGE TRIAL WITH RECOMBINANT ANTIGENS.**

(a) Vaccination protocol

The purified 21B4-309 fusion protein was supplied for vaccination trials. Twelve to fifteen months old Bos taurus British Breed steers were purchased from an area known to be free from Boophilus microplus, the tick vector for B. bovis and B. bigemina in Australia. The sera of the animals were then screened by both ELISA and IFA to determine that they contained no detectable antibodies to either Babesia species. The susceptible animals were divided into two groups, each of seven cattle.

Group 1, the control group, received 1 ml normal saline and 1 ml of FCA as a water-in-oil emulsion by subcutaneous injection on days 0 and 28.

Group 2 received -150µg of B. bovis native protein) in 1 ml of normal saline and 1 ml of Freunds Complete Adjuvant (FCA) as a water-in-oil emulsion by subcutaneous injection on days 0 and 28.

(b) Analysis of serum samples

Sera from all animals collected on weeks 1, 4, 6, 8 (day of challenge) were tested by ELISA and ranked against the optical density of 1.0 obtained by a B. bovis - positive refernece serum when tested against native B. bovis antigen (Waltisbuhl et al. 1987, Parasitology Research, 73:126-131). Further, after prior absorption of the sera with recombinant GST, sera from each animal was tested by ELISA against the recombinant antigen. At week 8 the sera from group 2 showed no significant antibody response to B. bovis native antigen (0.13 ± 0.05 OD), but a strong antibody response against 21B4-309 recombinant antigen (0.83 ± 0.09 OD).

(c) Challenge

The animals were challenged on Week 8 with a virulent heterologous challenge of 1 x $10^6$ erythrocytes infected with B. bovis (Lismore isolate). Parasitaemias for Groups 1 and 2 are shown (Figure 13). In group 1, individual animals showed parasitaemias from day 3. Temperature increases were moderate and PCV falls were moderate to severe. The mean maximum parasitaemia occurred on Day 8 after challenge and four animals were treated (Day 8 after challenge, 1 animal treated; Day 9 after challenge, 2 animals treated; Day 10 after challenge, 1 animal treated). Two animals showed only mild symptoms of clinical babesiosis. Individual animals in group 2 developed patent parasitaemias from Day 4 after challenge. Mean maximum parasitaemias occurred on Days 8 and 9 after challenge and showed a four-fold decrease in parasitaemia compared with group 1.

TABLE 1

| Results of IFA screening and designation of isotype of monoclonal antibodies secreted by hybridomas raised against the protective protease-containing fraction. | | |
|---|---|---|
| Monoclonal Antibody | Specificity of Staining | Isotype |
| T21B4 | parasite only | $IgG_2b$ |
| T10A1 | all cells | IgM |
| T16C6 | all cells | IgM |
| T16C6 | non-specific | IgM |
| T5D2 | all cells | IgM |

The invention also includes within its scope the aforesaid monoclonal antibody having the characteristic staining pattern as shown in FIG 4. FIG 4 shows that the monoclonal antibody stains an organelle(s) rhoptries which one located at the apical end of the parente.

It will be appreciated from the foregoing that the invention includes within its scope the sequences described previously. The invention includes within its scope the sequences described previously. The invention also includes within its scope sequences substantially homologous thereto (ie. sequences having greater than 40% homology over a length of 100 nucleotides or longer in the case of a DNA sequence and sequences having greater than 40% homology over a length of 30 amino acids or greater in the case of a protein). The term "substantially homologous thereto" may also include within its scope DNA sequences showing cross hybridisation with the DNA sequences described previously under standard hydridisation conditions.

A sample of the hybridoma corresponding to monoclonal antibody T21B4 was lodged at European Collection of Animal Cell Cultures, Ponton Down, Salisbury, Wiltshire, United Kingdom on November 22 1991 and since has been allocated a provisional accession number 91112212.

The clones Babesia bovis λgt11 1.1 and Babesia bovis λgt10 V.I.I.I.I were deposited at the Australian-Government Analytical Laboratories (AGAL) P.O. Box 385, Pymble, New South Wales, Australia 2073 on December 12 1991 and were allocated accession numbers N91/80846 and N91/80847 respectively. The plasmid pX309 was lodged at AGAL on November 12 1991 and was allocated accession number N91/80848.

It will also be appreciated that the invention includes within its scope not only polypeptides previously described which are useful as antigens but also vaccines which include the antigens as well as a suitable adjuvant. Appropriate adjuvants may include Freunds Complete Adjuvant, Freunds Incomplete Adjuvant, QuilA and other saponins or immunostimulating complexes (ISCOMS).

## Claims

**1.** A process for preparation of monoclonal antibody T21B4 which includes the following steps:

(i) fusing mouse splenocytes with an antigenic fraction derived from a lysate of Babesia bovis infected erythrocytes which have been subjected to further purification to remove contaminants including oxy-haemoglobin and from which a protease fraction hag been isolated following sodium dodecyl sulphate electrophoresis.

(ii) screening hybridomas obtained after step (i) with infected erythrocytes to locate hybridomas which stained the parasite in infected cells and thereafter obtaining a monoclonal antibody of isotype $IgG_2b$; and

(iii) purifying the monoclonal antibody extract of interest by passing a crude extract obtained after step (ii) which binds to isotype $IgG_2b$ followed by subsequent elution.

**2.** A monoclonal antibody T21B4 which is of $IgG_2b$ isotype and characterised by IMMUNOGOLD staining wherein granular staining of rhoptries organelles at the apical end of Babesia bovis in infected erythrocytes.

**3.** A monoclonal antibody T21B4 derived from hybridoma 91112212 lodged at the European Collection of Animal Cell Cultures.

4. An antigen inducing protective immunity against homologous or heterologous challenge with Babesia bovis which is immunoreactive with a monoclonal antibody recognising a protein located within rhoptries organelles at the apical end of Babesia bovis in infected erythrocytes.

5. An antigen immunoreactive with monoclonal antibody T21B4 derived from hybridoma 91112212 lodged at the European collection of Animal Cell Cultures.

6. A method of preparation of a native antigen 21B4 reactive with monoclonal antibody T21B4 as claimed in claim 5 which includes the steps of:
(i) passing lysed erythrocytes previously infected with Babesia bovis through an affinity column to which monoclonal antibody T21B4 was previously bound; and
(ii) eluting from the column 21B4 antigen.

7. A method of preparation of DNA fragments which encode protective component(s) of 21B4 antigen and corresponding polypeptides which includes the steps of:
(i) screening a B. bovis genomic or cDNA library with the oligonucleotide probe(s) prepared in step (iv).
(vi) identifying positive clone(s); and
(vii) isolating the DNA corresponding to the positive clone(s).

8. A method of preparation of DNA fragments which encode protective component(s) of 21B4 antigen and corresponding polypeptides which includes the steps of:
(i) screening a B. bovis genomic or cDNA library with T21B4 monoclonal antibody;
(ii) identifying positive clone(s); and
(iii) isolating the DNA corresponding to the positive clone(s).

9. Oligonucleotides having the structures depicted in FIG 7.

10. A DNA sequence as depicted in FIG 6 and sequences structurally homologous thereto.

11. A polypeptide having the sequence depicted in FIG 6 and sequences structurally homologous thereto.

12. A DNA molecule having the structure depicted in FIG 8.

13. A DNA sequence as depicted in FIG 9 and structures substantially homologous thereto.

14. A polypeptide having the structure depicted in FIG 9 and sequences structurally homologous thereto.

15. A DNA sequence as depicted in FIG 10 and sequences structurally homologous thereto.

16. A polypeptide having the sequence depicted in FIG 10 and sequences structurally homologous thereto.

17. A DNA sequence as depicted in FIG 11 and sequences structurally homologous thereto.

18. A polypeptide having the sequence depicted in FIG 11 and sequences structurally homologous thereto.

19. A DNA sequence as depicted in FIG 11 corresponding to the 309 fragment and sequences structurally homologous thereto.

20. A polypeptide as depicted in FIG 11 corresponding to the 309 fragment and sequences structurally homologous thereto.

21. An antigen derived from B. bovis clone λgt10 V I.I.I.I. deposited at the AGAL depositary under accession number N91/80846.

22. An antigen derived from B. bovis clone λgt11 1.1 and deposited at the AGAL depositary under accession number 91/80847.

**23.** An antigen derived from plasmid pX309 deposited at the AGAL depositary under accession number N91/80848.

**24.** A vaccine containing the antigen of claim 4 together with an adjuvant

**25.** A vaccine containing the antigen of claim 5 together with an adjuvant.

**26.** A vaccine containing the polypeptide of claim 11 together with an adjuvant.

**27.** A vaccine containing the polypeptide of claim 15 together with an adjuvant.

**28.** A vaccine containing the polypeptide of claim 17 together with an adjuvant.

**29.** A vaccine containing the polypeptide of claim 19 together with an adjuvant.

**30.** A vaccine containing the polypeptide of claim 21 together with an adjuvant.

**31.** A vaccine containing the antigen of claim 22 together with an adjuvant.

**32.** A vaccine containing the antigen of claim 23 together with an adjuvant.

**33.** A vaccine containing the antigen of claim 24 together with an adjuvant.

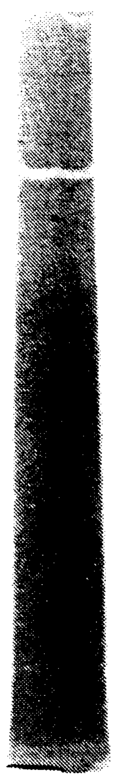

FIGURE 1. Substrate-containing SDS-PAGE of material eluted from T21B4 affinity column. Area with no stain represents antigen with proteolytic activity with a molecular weight estimated at 68-70kD.

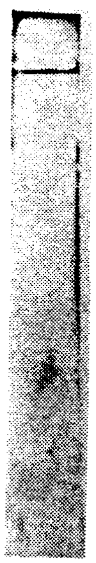

FIGURE 2. Silver stained SDS-PAGE of material eluted from T21B4 affinity column. The band visible corresponds to the band present in the substrate gel depicted in figure 1.

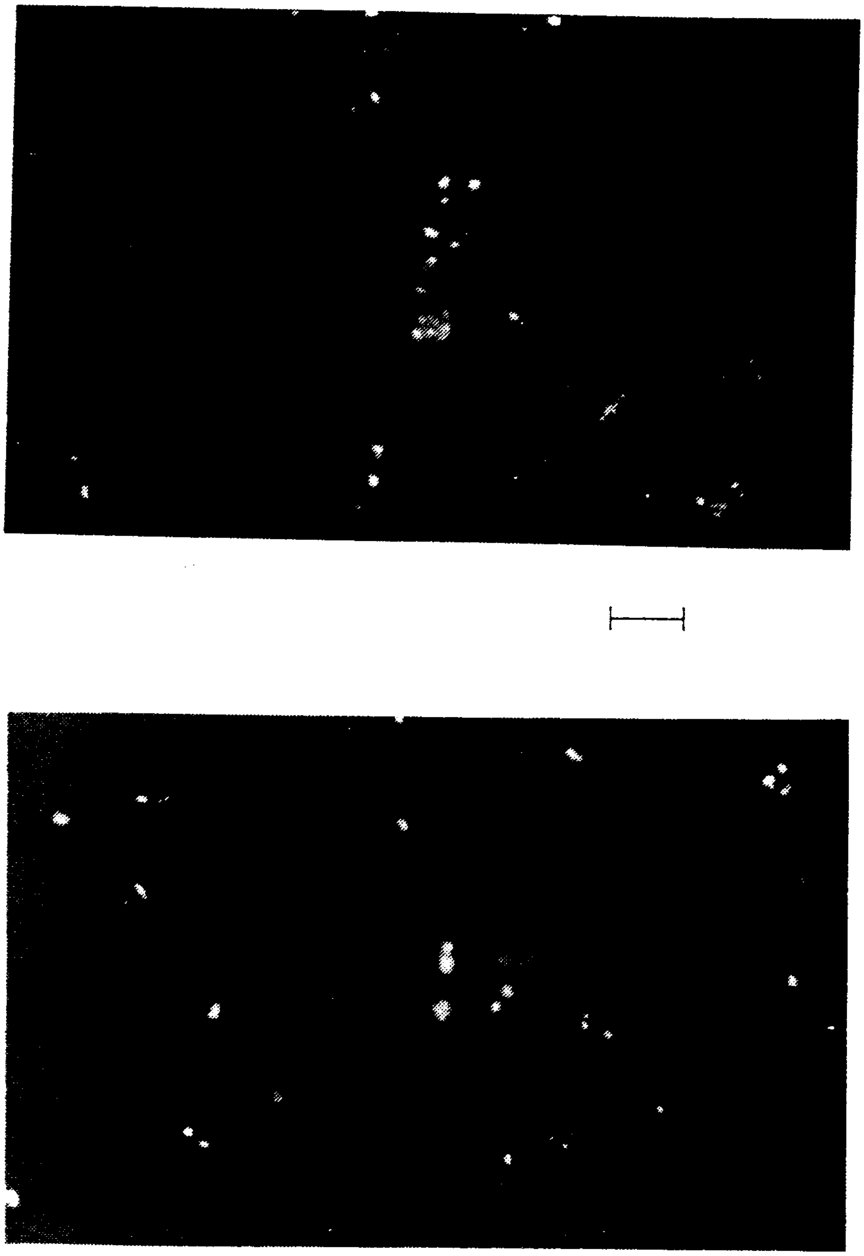

FIGURE 3 a, b. Bovine erythrocytes infected with *Babesia bovis* stained with mouse monoclonal antibody T21B4 and FITC labelled rabbit antiserum to mouse IgG. Granules at anterior of parasite and some infected erythrocytes are stained. (Bar=10μm).

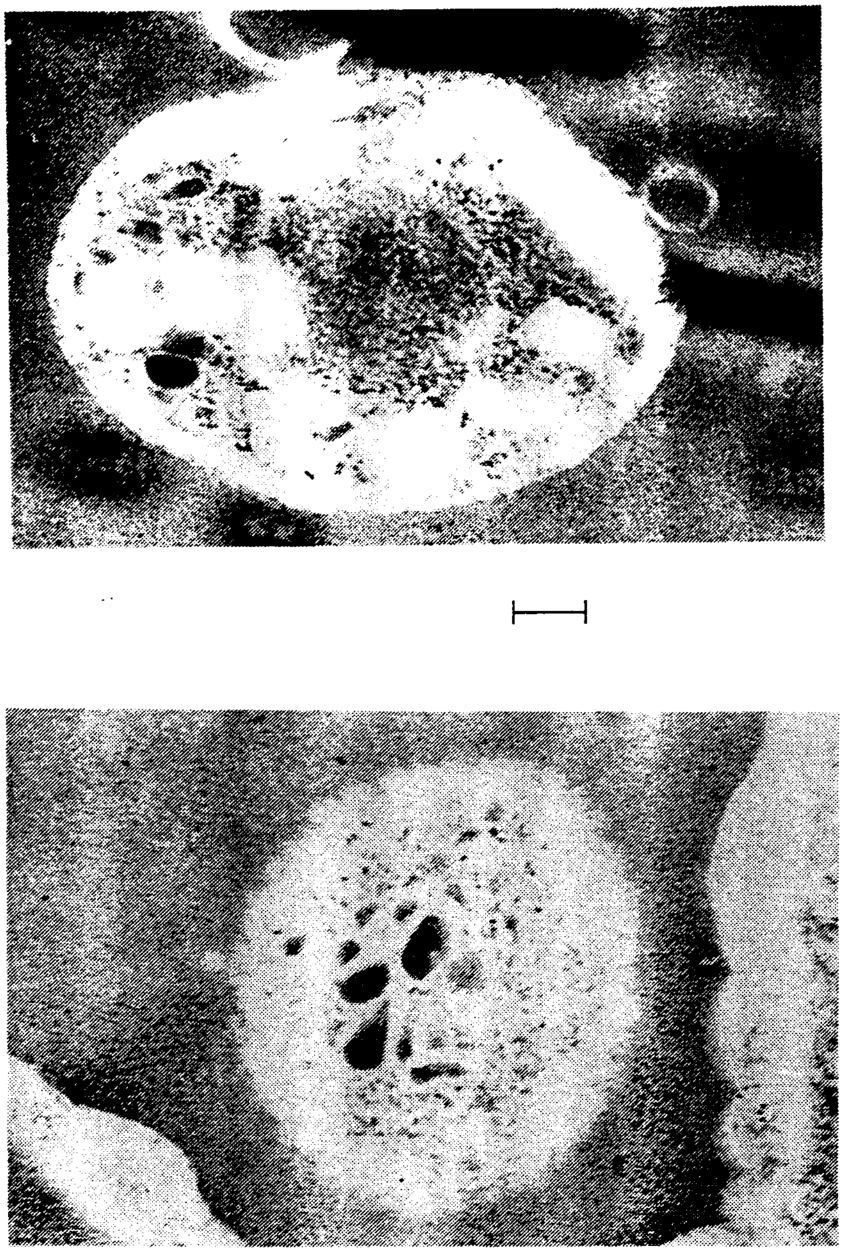

FIGURE 4 a, b. Transmission electron micrograph of cerebral cortex from a bovine castrated adult male infected acutely with *Babesia bovis* stained with mouse monoclonal antibody T21B4 and rabbit antiserum to mouse IgG and Protein A Gold (9nm). Rhoptries at anterior of parasite are stained intensely. Infected erythrocyte is stained weakly. (Bar=0.5μm).

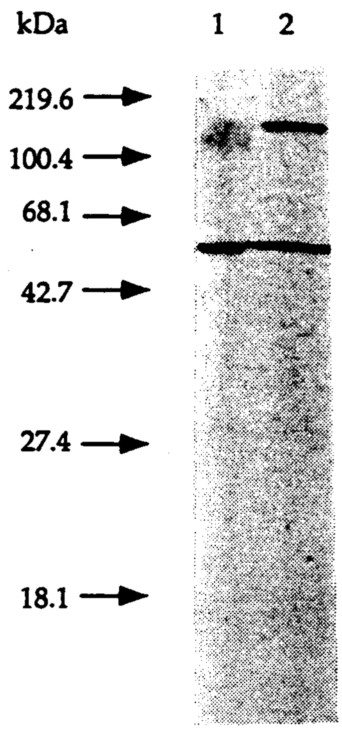


FIGURE 5. Western blot of SDS-PAGE of a non-recombinant λgt11 ß-galactosidase lysogen (lane 1) and clone I.1 recombinant ß-galactosidase lysogen (lane 2) probed with the T21B4 Mab. This bound to the recombinant ß-galactosidase fusion protein clearly (at about 116kDa) as well as to an *E. coli* protein running at about 60 kDa.

FIGURE 6. Complete cDNA sequence of the *Eco*RI insert from the plasmid pT#1 originally inserted in the clone *B. bovis* lambda gt11 I.1. The GGAATTCC - linker has been removed from the 5′ end, the *Eco*R I site at the 3′ end is genuine.

```
AG AAC ATT GGC CAA CCC ACC AAG GAG TTT TTC AGG GAA GCT CCC CAA GCC  50
   Asn Ile Gly Gln Pro Thr Lys Glu Phe Phe Arg Glu Ala Pro Gln Ala
   405                 410                 415                 420

ACT AAG CAC TTC TTA GAC GAG AAT ATT GCT CAA CCT ACT AAA GAA TTT  98
Thr Lys His Phe Leu Asp Glu Asn Ile Ala Gln Pro Thr Lys Glu Phe
                425                 430                 435

TTC AAG GAT GTC CCT CAA GTT ACC AAG AAA GTC TTG AGT GAG AAC ATT 146
Phe Lys Asp Val Pro Gln Val Thr Lys Lys Val Leu Ser Glu Asn Ile
            440                 445                 450

                        EcoRI
GCT CAA CCA ACT AAG GAA TTC  167
Ala Gln Pro Thr Lys Glu Phe
        455
```

FIGURE 7. DNA sequence of the synthetic oligonucleotides.

Sequence of 21B4.1 synthetic oligonucleotide:
5′ — CCAAGAAAGTCTTGAGTGAG — 3′

Sequence of 21B4.2 synthetic oligonucleotide:
5′ — AACTCCTTGGTGGGTTGCCC — 3′

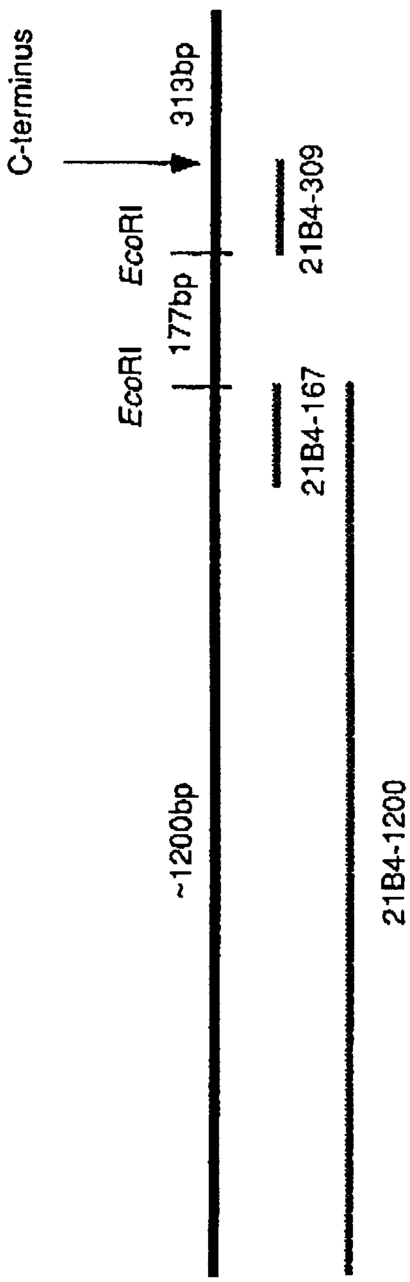


FIGURE 8. Organisation of lambda gt 10 clone V 1.1.1.1

FIGURE 9. Partial DNA sequence of the 1200 bp *Eco*RI fragment from the cDNA clone *B. bovis* lambda gt10 V 1.1.1.1 inserted into plasmid pT#13. This sequence was determined from the T7 end of the clone. The 5' end *Eco*RI site is a linker introduced during the cloning procedures. Amino acid residues are numbered according to the amino acid sequence of the complete 21B4 protein.

```
EcoRI
GGAATTCCC GCT CCA GCT GAA GTG GTA GGT GAT TTA ATC TCC ACA TTG  48
          Ala Pro Ala Glu Val Val Gly Asp Leu Ile Ser Thr Leu
                      35                  40

GAA ACA GCT GAT ACT TTG ATG ACT CTC CGT GAC CAC ATG CAC AAC ATT  96
Glu Thr Ala Asp Thr Leu Met Thr Leu Arg Asp His Met His Asn Ile
45                  50                  55                  60

ACT AAG GAT ATG AAA CAC GTT TTG AGC AAT GGT CGT GAG CAG ATT GTA 144
Thr Lys Asp Met Lys His Val Leu Ser Asn Gly Arg Glu Gln Ile Val
                65                  70                  75

AAT GAT GTT TGC TCT AAT GCT CCT GAG GAC TCC AAC TGT CGT GAG GTA 192
Asn Asp Val Cys Ser Asn Ala Pro Glu Asp Ser Asn Cys Arg Glu Val
            80                  85                  90

GTT AAC AAC TAT GCT GAC CGT TGT GAA ATG TAC GGA TGC TTC ACG ATT 240
Val Asn Asn Tyr Ala Asp Arg Cys Glu Met Tyr Gly Cys Phe Thr Ile
        95                  100                 105

GAC AAT GTC AGA TAT CCG TTG TAC CAA GAG TAC CAA CCT CTA         282
Asp Asn Val Arg Tyr Pro Leu Tyr Gln Glu Tyr Gln Pro Leu
    110                 115                 120
```

FIGURE 10. Partial DNA sequence of the 1200 bp *EcoRI* fragment from the cDNA clone *B bovis* lambda gt10 V 1.1.1.1 inserted in the plasmid pT#13. This sequence was determined from a deletion of part of the T7 end of the insert. Amino acid residues are numbered according to the amino acid sequence of the complete 21B4 protein.

```
AAC AAG GTA CTT TAT ATG GCT ACC ATG GAC TAC AAG ACT TAT TTG ACA   48
Asn Lys Val Leu Tyr Met Ala Thr Met Asp Tyr Lys Thr Tyr Leu Thr
                195                 200                 205

GTA AAC AGT ATG AAC GCC ANN TTC TTC AAC AGA TTC AGC TTT ACT ACA   96
Val Asn Ser Met Asn Ala Xxx Phe Phe Asn Arg Phe Ser Phe Thr Thr
            210                 215                 220

AAG ATA TTC AGT NNT CGT ATT AGG CAA ACA TTG AGT GAT ATC ATC AGG 144
Lys Ile Phe Ser Xxx Arg Ile Arg Gln Thr Leu Ser Asp Ile Ile Arg
        225                 230                 235

TGG AAT GTT CCT GAA GAT TTT GTA AG 170
Trp Asn Val Pro Glu Asp Phe Val
    240                 245
```

FIGURE 11. Complete DNA sequence of the *EcoRI* cDNA inserts from the clone *B. bovis* lambda gt10 V 1.1.1.1 inserted in plasmids pT#10 and pT#5. The sequence of the 309 fragment from pT#5 is highlighted by underlining. The final *EcoRI* site was introduced during the cloning procedures. Amino acid residues are numbered according to the amino acid sequence of the complete 21B4 protein.

```
EcoRI
GAA TTC CTT AGG GAG GTT CCT CAT ACT ACC ATG AAA GTC TTG AAT GAA   48
Glu Phe Leu Arg Glu Val Pro His Thr Thr Met Lys Val Leu Asn Glu
        460                 465                 470

AAC ATT GCT CAA CCT GCC AAG GAA ATC ATA CAT GAG TTT GGT TCA GGC   96
Asn Ile Ala Gln Pro Ala Lys Glu Ile Ile His Glu Phe Gly Ser Gly
    475                 480                 485

GCC AAG AAT TTC ATT TGG GCA GCC CAT GAA GGC ACT AAG CAG TTC TTA  145
Ala Lys Asn Phe Ile Trp Ala Ala His Glu Gly Thr Lys Gln Phe Leu
490                 495                 500                 505

                                        EcoRI
AAC GAA ACT GTT GGC CAA CCT ACA AGG GAA TTC CTT AAC GGN GCT TTA  192
Asn Glu Thr Val Gly Gln Pro Thr Arg Glu Phe Leu Asn Gly Ala Leu
                510                 515                 520

GAA ACT ACT AAA GAC GCA TTG CAC CAT CTG GGT AAA TCA TCA GAA GAA  241
Glu Thr Thr Lys Asp Ala Leu His His Leu Gly Lys Ser Ser Glu Glu
            525                 530                 535

GCC AAC ATT TAT GAT GCC ACG GAA AAT ACC ACT GAG GCT AAC GAC TCC  288
Ala Asn Ile Tyr Asp Ala Thr Glu Asn Thr Thr Glu Ala Asn Asp Ser
        540                 545                 550

ACT ACT TCC AAC GGT GAA GAC ACC GCC GGA TAC CTC TGATGA GATGCGTTGA  340
Thr Thr Ser Asn Gly Glu Asp Thr Ala Gly Tyr Leu
    555                 560                 565

TAATGGCACA AACTCAACAA ATGATGTATC GTCATCTGAT CCATCGGTTT TCAATATTGT  400

ATTGGATGCA ATATCTGAAT GTATATGATG CGACAGTTTC CATCATCGGG TGCCGAATCG  460

                    EcoRI
TAACTCTTAA AACACCGGAA TTC  483
```

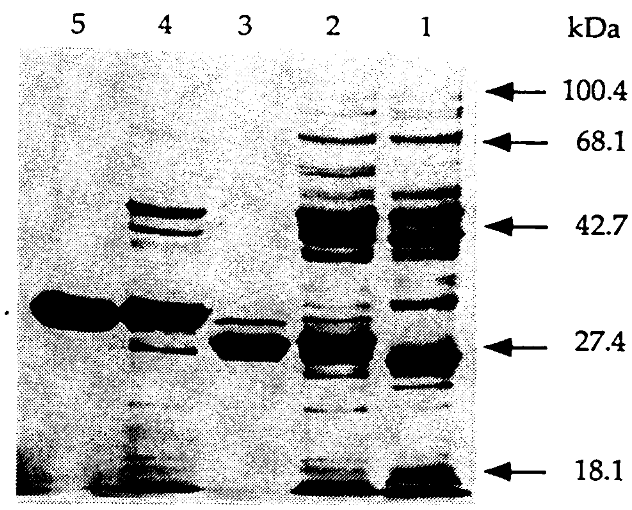


FIGURE 12. SDS-PAGE of glutathione-*S*-transferase fusion proteins 21B4-167 and 21B4-309, stained with Coomassie Blue R-250.

1: crude supernatant of pGEX-1 native glutathione-*S*-tranferase
2: crude supernatant of 21B4-167 glutathione-*S*-transferase fusion protein
3: affinity purified 21B4-167 glutathione-*S*-transferase fusion protein
4: crude supernatant of 21B4-309 glutathione-*S*-transferase fusion protein
5: affinity purified 21B4-167 glutathione-*S*-transferase fusion protein

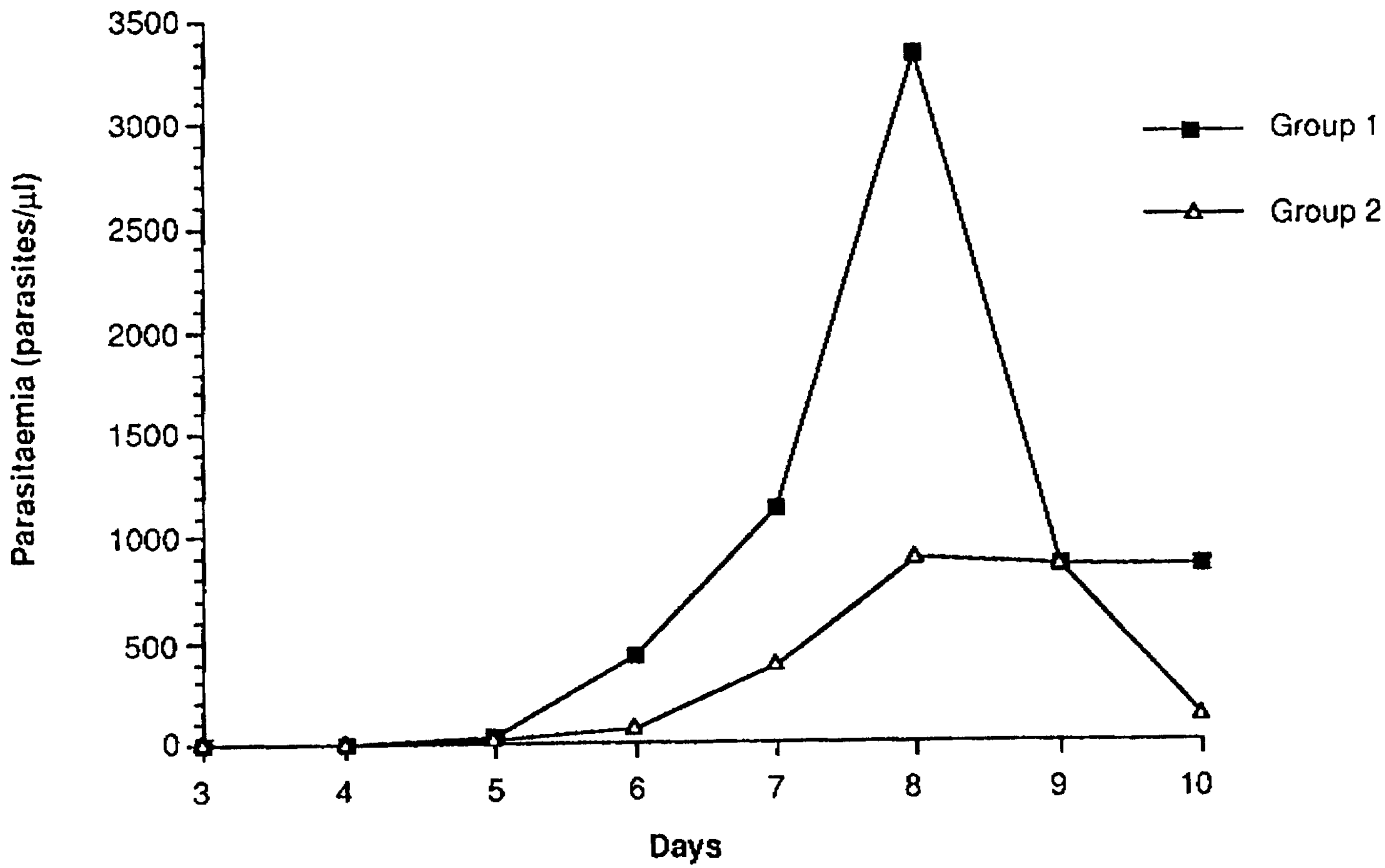


FIGURE 13. Profile of parasitaemia data for group 1 (controls) and group 2 (21B4-309 vaccinates)